(19) Europäisches Patentamt
European Patent Office
Office européen des brevets

(11) **EP 2 620 772 A1**

## (12) EUROPEAN PATENT APPLICATION

(43) Date of publication:
**31.07.2013 Bulletin 2013/31**

(51) Int Cl.:
***G01N 33/574*** *(2006.01)*

(21) Application number: **12152404.5**

(22) Date of filing: **25.01.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **APP Latvijas Biomedicinas petijumu un studiju centrs**
**1067 Riga (LV)**

(72) Inventors:
• **Line, Aija**
 **LV-1067 Riga (LV)**

• **Kalnina, Zane**
 **LV-1067 Riga (LV)**
• **Zajakins, Pavels**
 **LV-1067 Riga (LV)**
• **Silina, Karina**
 **LV-1067 Riga (LV)**

(74) Representative: **Fortuna, Jevgenijs**
**Foral Patent Law Office**
**P.O. Box 98**
**1050 Riga (LV)**

(54) **Gastric cancer biomarkers and methods of use thereof**

(57) The invention generally relates to biomarkers associated with cancer and methods and compositions for the detection, diagnosis, prognosis, prediction of therapy outcome and monitoring of the progression of cancer, in particular, stomach cancer. A peptide identified in SEQ ID 1, which can be used in biomarker detection panels adapted for the diagnosis and/or prognosis of cancer is offered. According to another aspect of the invention, a set of 20 antigenic peptides comprising amino acid sequences of SEQ ID Nos. 1 to 20 can be used in biomarker detection panel adapted for the diagnosis, prognosis, and/or prediction of therapy outcome of gastric cancer. This autoantibody test is the first test that can detect early stage gastric cancer with a similar sensitivity as late stage cancer and reaches a substantially higher specificity than any other gastric cancer serum marker known to date. It can detect gastric cancer of various histological types with equal accuracy and distinguish cancer from gastric inflammatory diseases with the highest precision known to date.

Fig. 1

SEQUENCE LISTING

<110> LATVIJAS BIOMEDICINAS PETIJUMU UN STUDIJU CENTRS

<120> GASTRIC CANCER BIOMARKERS AND METHODS OF USE THEREOF

<130> LAP 2/2002

<160> 20

<170> BiSSAP 1.0

<210> 1
<211> 34
<212> PRT
<213> Artificial Sequence

<220>
<221> SOURCE
<222> 1..34
<223> /mol_type="protein"
       /note="Generated by the translation of POP5 mRNA in Frame 3"
       /organism="Artificial Sequence"

<400> 1
Ser Ile Ser Leu Cys Gly Gln Leu Phe Pro Ser Ser His Thr Trp Arg
1               5                   10                  15
Thr Lys Asp Thr Val Thr His Ala Phe Ser Thr His Tyr Met Trp Glu
            20                  25                  30
Val Gln

<210> 2
<211> 105
<212> PRT
<213> Homo sapiens

<220>
<221> SOURCE
<222> 1..105
<223> /mol_type="protein"
       /organism="Homo sapiens"

<400> 2
Ala Met Gln Ala Glu Gly Arg Gly Thr Gly Gly Ser Thr Gly Asp Ala
1               5                   10                  15
Asp Gly Pro Gly Gly Pro Gly Ile Pro Asp Gly Pro Gly Gly Asn Ala
            20                  25                  30

```
Gly Gly Pro Gly Glu Ala Gly Ala Thr Gly Gly Arg Gly Pro Arg Gly
        35                  40                  45
Ala Gly Ala Ala Arg Ala Ser Gly Pro Arg Gly Gly Ala Pro Arg Gly
        50                  55                  60
Pro His Gly Gly Ala Ala Ser Ala Gln Asp Gly Arg Cys Pro Cys Gly
65                  70                  75                  80
Ala Arg Arg Pro Asp Ser Arg Leu Pro Glu Leu His Ile Thr Met Pro
                85                  90                  95
Phe Ser Ser Pro Met Glu Ala Glu Leu
                100                 105


<210> 3
<211> 180
<212> PRT
<213> Homo sapiens

<220>
<221> SOURCE
<222> 1..180
<223> /mol_type="protein"
      /organism="Homo sapiens"


<400> 3
Gly Trp Ser Gly Pro Phe Gly His Gln Gly Pro Arg Ala Ala Gly Ser
1               5                   10                  15
Arg Glu Pro Pro Leu Cys Phe Lys Ile Lys Asn Asn Met Val Gly Val
                20                  25                  30
Val Ile Gly Tyr Ser Gly Ser Lys Ile Lys Asp Leu Gln His Ser Thr
        35                  40                  45
Asn Thr Lys Ile Gln Ile Ile Asn Gly Glu Ser Glu Ala Lys Val Arg
        50                  55                  60
Ile Phe Gly Asn Arg Glu Met Lys Ala Lys Ala Lys Ala Ala Ile Glu
65                  70                  75                  80
Thr Leu Ile Arg Lys Gln Glu Ser Tyr Asn Ser Glu Ser Ser Val Asp
                85                  90                  95
Asn Ala Ala Ser Gln Thr Pro Ile Gly Arg Asn Leu Gly Arg Asn Asp
                100                 105                 110
Ile Val Gly Glu Ala Glu Pro Leu Ser Asn Trp Asp Arg Ile Arg Ala
                115                 120                 125
Ala Val Val Glu Cys Glu Lys Arg Lys Trp Ala Asp Leu Pro Pro Val
        130                 135                 140
Lys Lys Asn Phe Tyr Ile Glu Ser Lys Ala Thr Ser Cys Met Ser Glu
145                 150                 155                 160
Met Gln Val Ile Asn Trp Arg Lys Glu Asn Phe Asn Ile Thr Cys Asp
                165                 170                 175
Asp Leu Lys Ser
                180


<210> 4
<211> 155
```

```
<212> PRT
<213> Homo sapiens

<220>
<221> SOURCE
<222> 1..155
<223> /mol_type="protein"
      /organism="Homo sapiens"


<400> 4
Met Ser Pro Asn Asp Lys Val Ile Met Phe Val Ser Gln Lys His Ile
1               5                   10                  15
Ala Asp Asp Leu Ser Ser Asp Phe Asn Ile Gln Gly Ile Ser Ala Glu
            20                  25                  30
Ser Leu His Gly Asn Ser Glu Gln Ser Asp Gln Glu Arg Ala Val Glu
            35                  40                  45
Asp Phe Lys Ser Gly Asn Ile Lys Ile Leu Ile Thr Thr Asp Ile Val
        50                  55                  60
Ser Arg Gly Leu Asp Leu Asn Asp Val Thr His Val Tyr Asn Tyr Asp
65                  70                  75                  80
Phe Pro Arg Asn Ile Asp Val Tyr Val His Arg Val Gly Tyr Ile Gly
                85                  90                  95
Arg Thr Gly Lys Thr Gly Thr Ser Val Thr Leu Ile Thr Gln Arg Asp
            100                 105                 110
Ser Lys Met Ala Gly Glu Leu Ile Lys Ile Leu Asp Arg Ala Asn Gln
            115                 120                 125
Ser Val Pro Glu Asp Leu Val Val Met Ala Glu Gln Tyr Lys Leu Asn
        130                 135                 140
Gln Gln Lys Arg His Arg Glu Thr Arg Ser Arg
145                 150                 155

<210> 5
<211> 176
<212> PRT
<213> Homo sapiens

<220>
<221> SOURCE
<222> 1..176
<223> /mol_type="protein"
      /organism="Homo sapiens"


<400> 5
Leu Ala Leu Ile Glu Val Asp Pro Asp Asp Ser Tyr Val Phe Val Asn
1               5                   10                  15
Thr Leu Asp Leu Thr Ser Glu Gly Cys Leu Ser Asp Glu Gln Gly Met
            20                  25                  30
Ser Gln Asn Arg Leu Leu Ile Leu Ile Leu Ser Ile Ile Phe Ile Lys
            35                  40                  45
Gly Thr Tyr Ala Ser Glu Glu Val Ile Trp Asp Val Leu Ser Gly Ile
```

Gly Val Arg Ala Gly Arg Glu His Phe Ala Phe Gly Glu Pro Arg Glu

Leu Leu Thr Lys Val Trp Val Gln Glu His Tyr Leu Glu Tyr Arg Glu

Val Pro Asn Ser Ser Pro Pro Arg Tyr Glu Phe Leu Trp Gly Pro Arg

Ala His Ser Glu Ser Ile Lys Lys Lys Val Leu Glu Phe Leu Ala Lys

Leu Asn Asn Thr Val Pro Ser Ser Phe Pro Ser Trp Tyr Lys Asp Ala

Leu Lys Asp Val Glu Glu Arg Val Gln Ala Thr Ile Asp Thr Ala Asp

Asp Ala Thr Val Met Ala Ser Glu Ser Leu Ser Val Met Ser Ser Asn


<210> 6
<211> 276
<212> PRT
<213> Homo sapiens


<220>
<221> SOURCE
<222> 1..276
<223> /mol_type="protein"
        /organism="Homo sapiens"


<400> 6
Ala Val Ser Val Ser Cys Ile Thr Tyr Leu Arg Gly Ile Phe Pro Glu

Cys Ala Tyr Gly Thr Arg Tyr Leu Asp Asp Leu Cys Val Lys Ile Leu

Arg Glu Asp Lys Asn Cys Pro Gly Ser Thr Gln Leu Val Lys Trp Met

Leu Gly Cys Tyr Asp Ala Leu Gln Lys Lys Tyr Leu Arg Met Val Val

Leu Ala Val Tyr Thr Asn Pro Glu Asp Pro Gln Thr Ile Ser Glu Cys

Tyr Gln Phe Lys Phe Lys Tyr Thr Asn Asn Gly Pro Leu Met Asp Phe

Ile Ser Lys Asn Gln Ser Asn Glu Ser Ser Met Leu Ser Thr Asp Thr

Lys Lys Ala Ser Ile Pro Leu Ile Arg Lys Ile Tyr Ile Leu Met Gln

Asn Leu Gly Pro Leu Pro Asn Asp Val Cys Leu Thr Met Lys Leu Phe

Tyr Tyr Asp Glu Val Thr Pro Pro Asp Tyr Gln Pro Pro Gly Phe Lys

Asp Gly Asp Cys Glu Gly Val Ile Phe Glu Gly Glu Pro Met Tyr Leu

```
Asn Val Gly Glu Val Ser Thr Pro Phe His Ile Phe Lys Val Lys Val
            180                 185                 190
Thr Thr Glu Arg Glu Arg Met Glu Asn Ile Asp Ser Thr Ile Leu Ser
            195                 200                 205
Pro Lys Gln Ile Lys Thr Pro Phe Gln Lys Ile Leu Arg Asp Lys Asp
        210                 215                 220
Val Glu Asp Glu Gln Glu His Tyr Thr Ser Asp Asp Leu Asp Ile Glu
225                 230                 235                 240
Thr Lys Met Glu Glu Gln Glu Lys Asn Pro Ala Ser Ser Glu Leu Glu
            245                 250                 255
Glu Pro Ser Leu Val Cys Glu Glu Asp Glu Ile Met Arg Ser Lys Glu
            260                 265                 270
Ser Pro Asp Leu
        275


<210> 7
<211> 109
<212> PRT
<213> Homo sapiens


<220>
<221> SOURCE
<222> 1..109
<223> /mol_type="protein"
        /organism="Homo sapiens"


<400> 7
Gly Ser Glu Gln Glu Ser Val Lys Glu Phe Leu Ala Lys Ala Lys Glu
1                 5                 10                  15
Asp Phe Leu Lys Lys Trp Glu Ser Pro Ala Gln Asn Thr Ala His Leu
            20                  25                  30
Asp Gln Phe Glu Arg Ile Lys Thr Leu Gly Thr Gly Ser Phe Gly Arg
        35                  40                  45
Val Met Leu Val Lys His Lys Glu Thr Gly Asn His Tyr Ala Met Lys
    50                  55                  60
Ile Leu Asp Lys Gln Lys Val Val Lys Leu Lys Gln Ile Glu His Thr
65                  70                  75                  80
Leu Asn Glu Lys Arg Ile Leu Gln Ala Val Asn Phe Pro Phe Leu Val
            85                  90                  95
Lys Leu Glu Phe Ser Phe Lys Asp Asn Ser Asn Leu Tyr
            100                 105


<210> 8
<211> 81
<212> PRT
<213> Homo sapiens


<220>
<221> SOURCE
<222> 1..81
```

```
<223>  /mol_type="protein"
       /organism="Homo sapiens"


<400>  8
Gly Ser Ala Gly Arg Gly Gly Cys Lys Glu Glu Ile Asn Asp Pro Lys
1                   5                   10                  15
Glu Ile Cys Val Gly Ser Ser Asp Thr Ser Tyr His Ser Gln Gln Lys
                20                  25                  30
Gln Ser Gly Asp Asn Gly Ser Gly Gly His Phe Ala His Pro Arg Glu
         35                  40                  45
Asp Arg Glu Asp Arg Gly Pro Arg Met Thr Lys Ser Ser Leu Gln Lys
      50                  55                  60
Leu Cys Lys Gln His Lys Leu Ser Ala Trp Ser His Pro Gln Phe Glu
65                  70                  75                  80
Lys



<210>  9
<211>  100
<212>  PRT
<213>  Homo sapiens

<220>
<221>  SOURCE
<222>  1..100
<223>  /mol_type="protein"
       /organism="Homo sapiens"


<400>  9
Glu Gln Glu Tyr Trp Arg Leu Val Glu Glu Lys Ile Gln Ala Leu Pro
1                   5                   10                  15
Thr Val Pro Val Thr Glu Glu His Val Gly Ser Gly Leu Glu Cys Pro
                20                  25                  30
Val Cys Lys Asp Asp Tyr Ala Leu Gly Glu Arg Val Arg Gln Leu Pro
         35                  40                  45
Cys Asn His Leu Phe His Asp Gly Cys Ile Val Pro Trp Leu Glu Gln
      50                  55                  60
His Asp Ser Cys Pro Val Cys Arg Lys Ser Leu Thr Gly Gln Asn Thr
65                  70                  75                  80
Ala Thr Asn Pro Pro Gly Leu Thr Gly Val Ser Phe Ser Ser Ser Ser
                85                  90                  95
Ser Ser Ser Ser
         100


<210>  10
<211>  37
<212>  PRT
<213>  Homo sapiens
```

```
<220>
<221> SOURCE
<222> 1..37
<223> /mol_type="protein"
      /organism="Homo sapiens"


<400> 10
Gly Thr Pro Trp Asn Glu Asp Cys Gly Lys Glu Lys Pro Glu Glu Ile
1               5                   10                  15
Gln Asp Pro Ala Ala Leu Thr Ser Asp Ala Glu Gln Pro Ser Gly Phe
            20                  25                  30
Thr Phe Ser Phe Phe
            35


<210> 11
<211> 7
<212> PRT
<213> Homo sapiens


<220>
<221> SOURCE
<222> 1..7
<223> /mol_type="protein"
      /organism="Homo sapiens"


<400> 11
Ser Leu Ile Glu Ser Leu His
1               5


<210> 12
<211> 32
<212> PRT
<213> Homo sapiens


<220>
<221> SOURCE
<222> 1..32
<223> /mol_type="protein"
      /organism="Homo sapiens"


<400> 12
Pro Gly Pro Gly Ala Glu Gly Ala Ser Pro Pro Arg Gly Gly Arg Gly
1               5                   10                  15
Trp Gln Gly His Ala Pro Ala His Gly Pro Gly Cys Ser Trp Gln Pro
            20                  25                  30


<210> 13
<211> 142
<212> PRT
<213> Homo sapiens
```

```
<220>
<221> SOURCE
<222> 1..142
<223> /mol_type="protein"
      /organism="Homo sapiens"


<400> 13
Met Gly Ala Pro Thr Leu Pro Pro Ala Trp Gln Pro Phe Leu Lys Asp
1               5                   10                  15
His Arg Ile Ser Thr Phe Lys Asn Trp Pro Phe Leu Glu Gly Cys Ala
                20                  25                  30
Cys Thr Pro Glu Arg Met Ala Glu Ala Gly Phe Ile His Cys Pro Thr
            35                  40                  45
Glu Asn Glu Pro Asp Leu Ala Gln Cys Phe Phe Cys Phe Lys Glu Leu
        50                  55                  60
Glu Gly Trp Glu Pro Asp Asp Asp Pro Ile Glu Glu His Lys Lys His
65                  70                  75                  80
Ser Ser Gly Cys Ala Phe Leu Ser Val Lys Lys Gln Phe Glu Glu Leu
                85                  90                  95
Thr Leu Gly Glu Phe Leu Lys Leu Asp Arg Glu Arg Ala Lys Asn Lys
                100                 105                 110
Ile Ala Lys Glu Thr Asn Asn Lys Lys Lys Glu Phe Glu Glu Thr Ala
            115                 120                 125
Lys Lys Val Arg Arg Ala Ile Glu Gln Leu Ala Ala Met Asp
        130                 135                 140


<210> 14
<211> 12
<212> PRT
<213> Artificial Sequence

<220>
<221> SOURCE
<222> 1..12
<223> /mol_type="protein"
      /note="Generated by the translation of the Homo sapiens cDNA clon
      e MGC:18304 IMAGE:4180012 (BC008618.1"
      /organism="Artificial Sequence"


<400> 14
Trp Leu Gln Glu Val Thr Val Glu Lys Leu Cys Val
1               5                   10


<210> 15
<211> 84
<212> PRT
<213> Homo sapiens


<220>
```

```
<221> SOURCE
<222> 1..84
<223> /mol_type="protein"
      /organism="Homo sapiens"


<400> 15
Thr Gln Tyr Glu Ser Gly Ser Met Asp Lys Ala Ala Asn Phe Ser Phe
1               5                   10                  15
Arg Asn Thr Leu Glu Gly Phe Ala Ser Pro Leu Thr Gly Ile Ala Asp
            20                  25                  30
Ala Ser Gln Ser Ser Met His Asn Ala Leu His Ile Tyr Met Asn Gly
        35                  40                  45
Thr Met Ser Gln Val Gln Gly Ser Ala Asn Asp Pro Ile Phe Leu Leu
    50                  55                  60
His His Ala Phe Val Asp Arg Cys Asp Leu Pro Gln Glu Glu Met Ser
65                  70                  75                  80
Ala Ser Tyr Thr




<210> 16
<211> 144
<212> PRT
<213> Homo sapiens

<220>
<221> SOURCE
<222> 1..144
<223> /mol_type="protein"
      /organism="Homo sapiens"


<400> 16
Glu Gln Arg His Pro Glu Glu Arg Glu Ile Pro Met Asp Pro Glu Ala
1               5                   10                  15
Gly Ala Glu Pro Leu Val Pro Ala Gln Asp Ala Ser Ser Gln Val Lys
            20                  25                  30
Arg Glu Asp Thr Leu Cys Val Arg Gly Gln Arg Gly Leu Glu Glu Arg
            35                  40                  45
Ala Ile Pro Thr Glu Ser Ile Thr Asp Ser Pro Ile Ser Ala Gln Asp
        50                  55                  60
Leu Leu Ser Arg Ile Lys Gln Glu Glu His Gln Cys Val Trp Asp Gln
65                  70                  75                  80
Gln Asp Leu Ala Asp Arg Asp Ile Pro Thr Asp Pro Asn Ser Glu Ser
                85                  90                  95
Leu Ile Ser Ala His Asp Ile Leu Ser Trp Ile Lys Gln Glu Glu Gln
            100                 105                 110
Pro Tyr Pro Trp Gly Pro Arg Asp Ser Met Asp Gly Glu Leu Gly Leu
            115                 120                 125
Asp Ser Gly Pro Ser Asp Ser Leu Leu Met Val Lys Asn Pro Pro Pro
        130                 135                 140
```

```
<210> 17
<211> 47
<212> PRT
<213> Homo sapiens

<220>
<221> SOURCE
<222> 1..47
<223> /mol_type="protein"
      /organism="Homo sapiens"

<400> 17
Cys Ala Gly Asp Glu Leu Thr Ala Asn Glu Leu Cys Arg Leu Gly Ala
1               5                   10                  15
Leu Asp Ile Leu Glu Glu Val Asn Val Ser Gly Thr Arg Lys Asn Lys
            20                  25                  30
Phe Ser Glu Ala Ala Tyr Asn Asn Cys Ser Ile Thr Ile Phe Pro
        35                  40                  45


<210> 18
<211> 115
<212> PRT
<213> Homo sapiens

<220>
<221> SOURCE
<222> 1..115
<223> /mol_type="protein"
      /organism="Homo sapiens"

<400> 18
Asn Asn Leu Lys Leu Ser Val Pro Asp Asn Arg Gln Leu Leu Glu Gln
1               5                   10                  15
Glu Ser Ile Met Lys Ala Gln Pro Gln His Glu Ser Leu Gly Asn Lys
            20                  25                  30
Gly Ile Ser Lys Thr Glu Ile Ser Asp Gln Glu Lys Glu Lys Glu Lys
        35                  40                  45
Glu Lys Ile Pro Phe Ile Leu Glu Gly Ser Leu Lys Ala Trp Lys Glu
    50                  55                  60
Glu Gln His Arg Leu Ala Glu Glu Glu Arg Leu Arg Glu Glu Lys Lys
65                  70                  75                  80
Ala Glu Lys Lys Gly Lys Glu Ala Gly Lys Lys Lys Gly Lys Asp Asn
                85                  90                  95
Ala Glu Lys Glu Asp Ser Arg Ser Leu Lys Lys Lys Ser Pro Tyr Lys
            100                 105                 110
Glu Lys Ser
        115


<210> 19
```

```
<211> 74
<212> PRT
<213> Homo sapiens

<220>
<221> SOURCE
<222> 1..74
<223> /mol_type="protein"
      /organism="Homo sapiens"

<400> 19
Glu Lys Pro Ser Lys Val Ser Leu Lys Ser Ser Asp Arg Gln Gly Ser
1               5                   10                  15
Asp Glu Glu Ser Val His Ser Asp Thr Arg Asp Leu Trp Thr Thr Thr
            20                  25                  30
Thr Leu Ser Gln Ala Gln Leu Asn Met Pro Leu Ser Glu Val Cys Glu
        35                  40                  45
Gly Phe Asp Glu Glu Gly Arg Asn Ile Ser Lys Thr Arg Gly Trp His
    50                  55                  60
Ser Pro Gly Arg Gly Ser Leu Asp Glu Gly
65                  70

<210> 20
<211> 14
<212> PRT
<213> Homo sapiens

<220>
<221> SOURCE
<222> 1..14
<223> /mol_type="protein"
      /organism="Homo sapiens"

<400> 20
Val Asp Val Asp Asp Asp Ile Trp Glu Asp Glu Val Glu Asp
1               5                   10
```

**Description**

**Technical field**

The invention generally relates to biomarkers associated with cancer and methods and compositions for the detection, diagnosis, prognosis, prediction of therapy outcome, and monitoring of the progression of cancer, in particular, stomach cancer.

**Background Art**

Despite the overall global decrease in incidence, gastric cancer (GC) remains the fourth most common type of cancer and the second most common cause of cancer-related death worldwide [1]. Radical surgery of the tumour is the only potentially curative treatment, but early detection of the disease is an absolute prerequisite for a successful outcome [2]. Gastric cancer is frequently undiagnosed until a relatively advanced stage, when treatment options are markedly limited [3]. The reasons include: (1) the symptoms of gastric cancer become a burden to the patient in a relatively advanced stage, (2) the major diagnostic approach of gastric cancer currently is gastroscopic or surgical biopsy, but its potential to diagnose the disease early is limited [4]. Besides that it is an invasive and traumatic procedure and is not applicable for regular screening of a wide asymptomatic population and/or risk groups (like patients of atrophic gastritis) [5].

In order to improve the success rate of gastric cancer treatment, it is necessary to develop novel non or minimally invasive diagnostic approaches capable to detect tumours at an early stage and that would be applicable for the screening of a wide population. There have been attempts to develop various cancer diagnostic and/or prognostic tests based on quantitative analyses of serum markers like CEA, CA 19-9, CA 72-4, pepsinogen, cytokeratins (CYFRA 21-1, TPA, TPS), HGC beta subunit and others. Pepsinogen is the only marker that has been used in diagnosis or screening programs. It reached 77% sensitivity and 73% specificity in gastric cancer screening in Japan - the country with the highest incidence of gastric cancer. None of the other serum markers are recommended for the use in diagnosis, prognosis or choice of therapeutic strategy of gastric cancer by the National Academy of Clinical Biochemistry (NACB) due to the low sensitivity (especially for early stage disease), low specificity and controversial data [5].

The detection of antibodies has successfully been used in clinical diagnosis of infectious diseases due to the high specificity and stability of antibodies in serum. A growing body of research has showed that antibodies are formed also in practically all cancer patients against structurally and/or quantitatively altered proteins in cancer cells - the so called cancer antigens, besides such antibodies can be detected in patients' sera at early disease stages [6]. Thus antibodies are very attractive biomarkers for the development of a minimally invasive (blood sample) diagnostic test for early cancer detection. Such experimental methods as SEREX (serological identification of tumour antigens by recombinant expression cloning) [7], provided the opportunity to identify proteins in cancer cells that are recognised by patients antibodies resulting in over 2000 various cancer antigens known to date [8]. However, several biological and immunological aspects have thus far hampered the development of a cancer diagnostic test based on antibody detection: (1) the antigen repertoire in each individual tumour can be very different, (2) in each patient antibodies can form against different antigens, and (3) antibodies partly overlap between patients of cancer and inflammatory diseases. Hence the sensitivity of a diagnostic test that is based on the detection of a single antibody (as in the case of infectious diseases) is very low. Besides that the repertoire of autoantibodies in gastric cancer patients has not been studied in detail and the identity of antigens eliciting cancer specific antibody formation at an early stage is elusive.

These problems can be solved by identifying a presentable set of antigens in gastric cancer and by using a high throughput technological platform to analyse autoantibodies in sera from healthy individuals, cancer patients and inflammatory disease patients against all of the antigens simultaneously to detect the truly cancer specific antibodies [9].

Previously there have been attempts to generate a set of antigens for the serodiagnosis of gastric cancer [18], however the known set contains other antigens and its diagnostic performance has not been validated in an independent sample set.

**Disclosure of Invention**

The aim of the invention is to offer an improvement for the early diagnosis of gastric cancer and to develop an effective gastric cancer diagnostic and/or prognostic test.

The set goal is achieved by identifying a set of antigens in gastric cancer. The goal is achieved by using a high throughput technological platform to analyse autoantibodies in sera from healthy individuals, cancer patients and gastric inflammatory disease patients against all of the antigens simultaneously to detect the truly cancer specific antibodies. In particular, the inventors have identified 1080 various cancer antigens by applying the phage display SEREX method, developed a microarray-based autoantibody profiling approach (antigen microchip) and a system for data processing and analyses that were used for the identification of gastric cancer specific autoantibodies. The obtained 1080 antigen microarray was screened with sera from gastric cancer and gastric inflammatory disease patients and healthy donors. As a result an artificial peptide having the following amino acid sequence SISLCGQLFPSSHTWRTKDTVTHAFSTHYMWEVQ (SEQ ID 1) was discovered as a novel gastric cancer specific antigen. Said peptide having a 13,6% sensitivity (frequency in stomach cancer) and a 100% specificity, can be used in biomarker detection panels, kits adapted for the diagnosis and/or prognosis, and/or prediction of therapy outcome of cancer. It can be used to significantly improve the specificity of the known cancer biomarkers. According to another aspect of the invention, a set of 20 antigenic peptides comprising amino acid sequences of SEQ ID Nos. 1 to 20 can be used in autoantibody detection panel adapted for the diagnosis and/or prognosis, and/or prediction of therapy outcome of gastric cancer. This autoantibody test reaches a considerably higher specificity than any other known gastric cancer serum marker known to date and can distinguish cancer from inflammatory diseases with high precision. Besides this is the first diagnostic test that can detect early stage cancer with a similar sensitivity as late stage cancer as well as cancers with various histological types. Further the inventors offer a method for an *in vitro* detection of antibodies against a peptide set forth in SEQ ID No. 1 or the set of peptides shown in SEQ. ID No.1 to 20, the method comprising bringing in contact individual's isolated biological sample (i.e. body fluid, including blood, plasma, serum, synovial fluid, urine, feces, interstital fluid, lymphatic fluid, saliva, sudor, spinal fluid, and/or lacrimal fluid) or a fraction of the said sample and the peptide of SEQ ID No. 1 or the said set of peptides and the detection of the presence of autoantibody that specifically recognises the peptide shown in SEQ. ID No.: 1 (or a variant, homologue, having the same properties as the parent sequence, or fragment thereof) or the set of peptides shown in SEQ. ID No.1 to 20 in the biological sample or its fraction (i.e. determine, whether antibodies from isolated body fluid are bound to the peptides).

Thus the invention offers an improvement for the early diagnosis of gastric cancer by providing a SEQ ID 1 antigen and the set of antigens that elicit autoantibody formation in gastric cancer patients with even an early stage disease. Besides the formation of IgG class antibodies is coordinated by antigen specific CD4+ T cells meaning that the presence of autoantibodies could reflect the functional state of the immune system and they could also be used for the prediction of immunotherapy efficiency and outcome. The peptides can be produced, purified and further used for the detection of autoantibodies in biological samples via any immunological test method, for example ELISA, Western blot, antigen microarrays and bead-based technologies like the Luminex technology and others.

*Explanation of the terms used in the description and claims.*

By "biomarker" a biochemical characteristic that can be used to diagnose, or to measure the progress of a disease or condition, or the effects of treatment of a disease or condition is meant. A biomarker can be, for example, the presence of a nucleic acid, protein, or antibody associated with the presence of cancer or another disease in an individual. The present invention provides biomarkers for stomach cancer that are antibodies present in the body fluids of subjects diagnosed with stomach cancer. The biomarker antibodies in the present invention are the autoantibodies displaying increased reactivity in individuals with stomach cancer. The autoantibodies can be detected with autoantigens, human proteins that are specifically bound by the antibodies.

As used herein, the word "protein" refers to a full-length protein, a portion of a protein, or a peptide. The term protein includes antibodies. Proteins can be produced via fragmentation of larger proteins, or chemically synthesized. As used herein, the term "peptide," "oligopeptide," and "polypeptide" are used interchangeably with protein herein and refer to a sequence of contiguous amino acids linked by peptide bonds. As used herein, the term "protein" refers to a polypeptide that can also include post-translational modifications that include the modification of amino acids of the protein and may include the addition of chemical groups or biomolecules that are not amino acid-based. The terms apply to amino acid polymers in which one or more amino acid residue is an analog or mimetic of a corresponding naturally occurring amino acid, as well as to naturally occurring amino acid polymers.

"Antibody" refers to a polypeptide ligand substantially encoded by an immunoglobulin gene or immunoglobulin genes, or fragments thereof, which specifically recognizes and binds a molecule or a region or domain of a molecule (an epitope). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well characterized fragments produced by digestion with various peptidases. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. An "autoantibody" is an antibody present in an individual that specifically recognizes a biomolecule present in the individual or its structural mimetic (mimotope - the mimetic of a specific epitope). Typically an autoantibody specifically binds a protein expressed by the individual, or a modified form thereof present in a sample from the individual. Autoantibodies are generally IgG antibodies that circulate in the blood of an individual, although the invention is not limited to IgG autoantibodies or to autoantibodies present in the blood.

As used herein, a "biomarker detection panel" or "biomarker panel" refers to a collection of biomarkers that are provided together for detection, diagnosis, prognosis, staging, or monitoring of a disease or condition, based on detection values for the set (panel) of biomarkers. The set of biomarkers is physically associated, such as by being packaged together, or by being reversibly or irreversibly bound to a solid support. For example, the biomarker detection panel can be provided, in separate tubes that are sold and/or shipped together, for example as part of a kit, or can be provided on a chip, membrane, strip, filter, or beads, particles, filaments, fibers, or other supports, in or on a gel or matrix, or bound to the wells of a multiwell plate. A biomarker detection panel can in addition or in the alternative be associated by a list, table, or program provided to a user or potential user that provides an internet address that provides computer-based linkage of the biomarker identities and information stored on a web site. A computer-based program can provide links between biomarker identities, information, and/or purchasing functions for a collection of biomarkers that make up a biomarker detection panel, based on the user's entered selections.

## Brief description of drawings

Fig. 1 shows autoantibody response against antigen clone No 509 (SEQ ID No. 1), where horizontal line indicates the serum positivity threshold; GC - gastric cancer patients, HD - healthy donors, K25 - gastric ulcer patients, K29 - chronic gastritis patients;
Figs. 2A and 2B show a diagnostic value of the 20 antigen set when comparing gastric cancer (GC) patients and healthy donors (HD).
Figs. 3A and 3B show a diagnostic value of the autoantibody test for the detection of gastric cancer of various stages (A) and for patients with gastric ulcer (K25) and gastritis (K29) (B).

There are the following steps in the identification of antigens that are eliciting cancer associated autoantibody formation:

1) obtaining RNA and cDNA from cancer tissues,
2) construction of cDNA expression library using the T7 phage display expression system,
3) immunoscreening of the recombinant phage library with patients' sera, isolation of serum positive clones and identification of the inserts,
4) production of phage displayed antigen microarray and screening with sera from cancer patients and healthy donors (training study),
5) microarray data processing and statistical analyses to determine the most significant cancer associated autoantibodies,
6) validation of the most significant autoantibodies using a focused phage displayed antigen microchip and a different set of gastric cancer patients' sera, sera from inflammatory disease patients and healthy donors.

*Samples and study populations.* All tissue and serum specimens were collected after the patients' informed consent was obtained in accordance with the regulations of Committee of Medical Ethics of Latvia and the ethical committee of the clinical partner organisations.

*Tissue samples.* Eleven gastric cancer tissue specimens were macroscopically dissected by a histopathologist during surgery at Latvian Oncology Center and stored in RNALater® (Applied Biosystems, USA) at -80°C till processing. Tissue sections were evaluated by an experienced pathologist and the diagnosis was established according to standard histopathological criteria. Total testis RNA was purchased from Applied Biosystems.

*Serum samples.* All cancer patients' serum samples were obtained before treatment and stored at -80°C till processing. 232 gastric cancer patients' serum samples were obtained from Latvian Oncology Center, 134 - from Clinic of Gastroenterology, Hepatology and Infectious Diseases, Germany. Serum samples from 313 age and gender matched cancer-free healthy individuals and 98 patients with gastritis and 52 patients with gastric ulcer were provided by the Genome Database of Latvian Population. Characteristics of the study population are provided in Table 1.

Table 1. Clinicopathological characteristics of the study population

| Variable | | Antigen discovery | Training set | Validation set |
|---|---|---|---|---|
| **GC patients** | | | | |
| Number of patients | | 27 (positive selection) | 100 | 239 |
| Age (mean (yr) $\pm$SD) | | 64.6 $\pm$9.8 | 68$\pm$13 | 67$\pm$13 |
| Sex: | Male | 16 | 59 | 141 |

(continued)

| Variable | Antigen discovery | Training set | Validation set |
|---|---|---|---|
| **GC patients** | | | |
| Female | 11 | 41 | 98 |
| Histological type (Lauren classification): | | | |
| Intestinal | 13 | 40 | 88 |
| Diffuse | 10 | 38 | 80 |
| Mixed | - | 9 | 17 |
| Data not available | 4 | 13 | 54 |
| Stage: I | 4 | 18 | 31 |
| II | 4 | 12 | 28 |
| III | 3 | 18 | 40 |
| IV | 5 | 20 | 42 |
| Data not available | 11 | 32 | 98 |
| Grade: 1 (well differentiated) | 1 | | 16 |
| 2 (moderately differentiated) | 7 | | 46 |
| 3 (poorly differentiated) | 8 | | 117 |
| 4 (undifferentiated) | 3 | | 10 |
| Data not available | 8 | 100 | 50 |
| Localization: Cardia | - | | 43 |
| Fundus | 2 | | - |
| Corpus | 4 | | 102 |
| Antrum/pylorus | 7 | | 49 |
| Total | 1 | | 3 |
| Data not available | 13 | 100 | 42 |
| *H. pylori* status*: HP+, CagA+ | | | 70 |
| HP+, CagA- | | | 23 |
| HP-, CagA- | | | 41 |
| Data not available | 27 | 100 | 105 |
| **Controls** | | | |
| Gastritis | - | - | 98 |
| Age (mean (yr) ±SD) | | | 68±10 |
| Sex: | | | |
| Male | | | 55 |
| Female | | | 43 |
| Gastric ulcer | - | - | 52 |
| Age (mean (yr) ±SD) | | | 65±10 |
| Sex: | | | |
| Male | | | 28 |

(continued)

| Controls | | | |
|---|---|---|---|
| Female | | | 24 |
| Cancer-free healthy controls | 5 (negative selection) | 100 | 213 |
| Age (mean (yr) ±SD) | NA | 70±5 | 71±5 |
| Sex: | | | |
| Male | 3 | 52 | 117 |
| Female | 2 | 48 | 96 |
| *, *H. pylori* status was serologically determined by analyzing anti.*H. pylori* IgG levels as described in Wex et al., 2010. [10] | | | |

*Construction of cDNA expression libraries.* Total RNA was extracted from 11 tumor tissue specimens using TRIzol reagent (Invitrogen) according to the manufacturer's instructions. mRNA was isolated from 150 $\mu$g commercial testis total RNA and 300 $\mu$g total RNA pooled from gastric cancer specimens obtained from several patients using Dynabeads mRNA purification kit (Invitrogen) and converted to cDNA using HindIII Random primers (5'-TTNNNNNN-3') (Novagen). Then cDNA was ligated to directional EcoRI and HindIII linkers, digested with the corresponding restriction enzymes, size fractioned by gel electrophoresis to isolate fragments of 200 - 1000 bp in length and then ligated into pre-digested previously prepared T7 Select 10-3b vectors (Novagen) according to the manufacturers protocol. The ligation mixtures were packaged *in vitro* using the T7 phage packaging extract from the above kit according to the manufacturers protocol and yielding cDNA expression libraries of $5\times10^6$ pfu and $8\times10^6$ pfu, respectively. The libraries were amplified once in IPTG-induced BLT5615 cells.

*Identification of serum-reactive antigens.* First, the recombinant phages recognised by healthy donor autoantibodies were removed (negative selection) by using G protein coupled magnetic beads and a serum pool from five healthy donors. Next the libraries were enriched with phage particles that express antigens that are recognised by sera from gastric cancer and inflammatory disease patients (positive selection) using G protein coupled magnetic beads and pools of sera from gastric cancer and gastritis patients. Sera were pre-absorbed with BLT5615 and T7 phage lysate coupled to CNBr-Sepharose 4B before adding to the phage libraries. The enriched libraries were immunoscreened with pre-absorbed sera from gastric cancer patients as described previously [9]. Briefly, BLT5615 cells grown in LB supplemented with 1xM9 salts, 0.4% glucose, 1mM $MgSO_4$ and carbenicillin (50$\mu$g/ml) to $OD_{600}$=0.5 and induced with IPTG for 30 min were infected with the phages and plated on LB/carbenicillin agar plates at density $\sim 10^3$pfu per 150 mm plate. After ~2h incubation at 37°C when the plaques reached ~1mm in diameter, plates were overlaid with Protan nitrocellulose (NC) filters (Whatman) and incubated for 1 h at 37°C. The filters were blocked with 5% (w/vol) milk powder in TBS, 0.05% Tween 20 for 1h, and then incubated overnight with 1:200 diluted patients' serum that has been preabsorbed with *E.coli*/ phage lysates immobilised on CNBr-Sepharose 4B. The serum-reactive clones were detected by incubating the filters with alkaline phosphatase conjugated anti-human IgG, Fcy specific secondary antibody (Pierce) and NBT/BCIP (Fermentas), isolated and purified to monoclonality. The inserts of serum-reactive phages were amplified by 35-cycle PCR using primers flanking the insert and 1 $\mu$l of phage solution as a template. PCR products were purified and sequenced using ABI Prism BigDye Terminator v3.1 cycle sequencing kit and 3130 Genetic Analyser (Applied Biosystems).

*Production and processing of phage displayed antigen microarrays. 1080* identified serum positive recombinant phage clones and 70 non-recombinant phage controls were used to create a phage displayed antigen microarray as described previously [9]. Briefly, all phages were simultaneously amplified to high titer (~$5\times10^8$-$1\times10^9$ pfu/$\mu$l) in *E. coli* BLT 5616 cells using 96 well culture plates (Whatman). The lysates were clarified by centrifugation, supplemented with 5% glycerol and 0.1%$NaN_3$ and arrayed in quadruplicates onto nitrocellulose-coated 2-pad FAST slides (Whatman) using a QArray Mini microarrayer (Genetix). The microarray slides were blocked with 5% (w/vol) milk powder in TBS, 0.05% Tween 20, incubated with 0.9 ml of 1:200 diluted patients' sera that were pre-absorbed with 15 $\mu$l of UV-inactivated *E.coli*- phage lysates, washed 4 times in TBS, 0.5% Tween 20 for 15 min, and then incubated with monoclonal anti-T7 tail fiber antibody (Novagen). After 3 washes in TBS, 0.5% Tween 20, the microarrays were incubated with Cy5 labelled goat anti-human IgG antibody (1:1500) and Cy3 labeled goat anti-mouse IgG antibody (1:3000) (Jackson ImmunoResearch) for 1 h, then washed thrice in TBS, 0.5% Tween 20, rinsed with distilled water and dried by centrifugation. A reference serum was included in each series of experiments. The arrays were scanned at 10 $\mu$m resolution in PowerScanner (Tecan) with 532 and 635 nm lasers, the results were recorded as TIFF files and the data were extracted using GenePix software. The obtained data were further analyzed using an ad hoc program composed in R language.

86 most significant antigens and 10 non-recombinant controls were used for the production of 96-feature antigen microarray, the selected phage clones were amplified from the low-titre stocks as described before, quality-controlled by PCR and spotted onto 16-pad FAST slides in duplicates and the arrays were processed as described above.

*Microarray data processing and statistical analysis.* For each spot the mean Cy5 and Cy3 signals were background subtracted, averaged between replicates, and the Cy5/Cy3 ratios were calculated for each antigen. Spots that did not pass the quality criteria (morphologically heterogeneous spots and spots that differed by more than 50% between replicates) were excluded from the analysis. A two-step normalization strategy was used for the fluorescent signal ratios in order to eliminate variations introduced by the custom production of microarrays and variable background intensities of different sera. At first, the values in each slide (each serum) were normalized by the median of the middle 80% of all measurements for each fluorescent channel separately. Then the distribution of data across an array was centred by equalizing the standard deviation of the middle 80% of values to 1. Next, for the inter-slide normalization, the Cy5 and Cy3 signal intensities for each spot were divided by the median of the middle 80% of the values for this spot in slides within one batch and the distribution was centred across slides in the batch. The highest and lowest 10% of the values were excluded from the SD calculation in order not to dismiss the outliers that may represent serum-positive antigens.

The cut-off value (T) for each antigen was calculated as follows: $T = mean(I_{HD}) + 3 \times SD(I_{HD})$, but not lower than the second highest IHD, where IHD is the signal intensities in healthy controls. Then the antigens were ranked, taking into account the signal intensity and frequency of reactivity with GC patient sera compared to healthy donor sera, using the following formula: $R_i = \left( \dfrac{\Sigma I_{GC_i}}{N_{GC_i}} \right) - 2 \left( \dfrac{\Sigma I_{HD_i}}{N_{HD_i}} \right).$

Finally, a score for each serum was calculated as follows: $S = \sum_{i=1}^{n} \sqrt{R_i} \times I_i$. The non-parametric Mann-Whitney U test was used to compare the serum scores between two independent groups of samples. The receiver operating characteristic (ROC) curve was constructed and the area under the curve (AUC) was calculated to evaluate the diagnostic performance of the serum scores. Leave one-out cross validation (LOOCV) as described by Laxman B et al, 2008 was used to validate the biomarker models to eliminate overestimated values (22). To define cut-off points on the ROC curves with the maximal sum of sensitivity and specificity Minimal misclassification cost term (MCT) approach (23) was used as follows:

$$MCT = (1 - prevalence) \times (1 - Sp) + \left( \frac{cost(FN)}{cost(FP)} \times prevalence \times \right)(1 - Se),$$ where the cost

(FN)/cost(FP) was set at 0.5.

*Selection of diagnostically most significant antigens.* In order to define autoantibodies with a potential diagnostic significance, the 1080-feature microarray was tested with a sample training set containing sera from 100 patients with gastric cancer of various histological types and stages and 100 age and gender matched healthy individuals (Table 1) and the microarray data was processed and analysed as described above. The obtained results showed that 360 of the analysed antigens had positive rating - i.e. the signal intensity and the frequency of autoantibodies among gastric cancer patients' sera are significantly higher than in the healthy controls. 78 best antigens were selected from the 360 antigens (Table 2) that could discriminate the cancer patients' sera from healthy control sera in the training set cohort with a similar efficiency as the 360 antigens with the positive rating. To determine the diagnostic value of these antigens, a focused microarray version was prepared containing these antigens and negative controls, and was tested with an independent serum set (the validation set) including sera from 239 GC patients at various stages and of various histological types, 213 healthy individuals with no history of cancer, 52 patients with peptic ulcer and 98 patients with acute or chronic gastritis (Table 1) and all the calculations were performed as described above.

*Description of the antigen set applicable for the diagnosis and/or prognosis of gastric cancer.* The inventors have used a custom made antigen database and clone collection, antigen microarray technology and an original approach of data statistical processing and have identified a set of 20 antigens that is applicable for the detection of antibodies in human serum for the diagnosis, prognosis and prediction of immunotherapy outcome of gastric cancer (Table 2).

Table 2. The set of most significant 20 antigens for the diagnosis and/or prognosis of gastric cancer.

| No. / Seq. ID | Clon | Gene symbol | Rating score | Sensitivity (Freq in GC), % | Specificity (100-Freq in HD), % | Odds ratio | Fisher exact t test p-value | Freq. in inflammatory diseases, % K25 | Freq. in inflammatory diseases, % K29 | Peptide sequence |
|---|---|---|---|---|---|---|---|---|---|---|
| 1* | 509 | | 13 144 | 1 13 6 | 1 00 0 | Inf | 000 006 7 | 0 | 0 | SISLCGQLFPSSHTWRTKDTVTHAFSTHYMWEVQ |
| 2 | 14 1 6 | CTAG2 | 19 73 61 | 1 7 2 5 | 9 9 5 3 | 4 1 3 4 | 8 4E 11 | 0 | 0 | AMQAEGRGTGGSTGDADGPGGPGIPDGPGGNAGGPGEAGATGGRGPRGAGAARASGPRGGAPRGPHGGAASAQDG RCPCGARRPDSRLPELHITMPFSSPMEAEL |
| 3 | 14 2 8 | DDX53 | 25 9 | 6 8 7 | 1 0 0 | Inf | 000 003 4 | 19 2 | 0 | GWSGPFGHQGPRAAGSREPPLCFKIKNNMVGVVIGYSGSKIKDLQHSTNTKIQIINGESEAKVRIFGNREMKAKAKAA IETLIRKQESYNSESSVDNAASQTPIGRNLGRNDIVGEAEPLSNWDRIRAAVVECEKRKWADLPPVKKNFYIESKATSC MSEMQVINWRKENFNITCDDLKS |
| 4 | 14 2 9 | DDX53 | 86 05 | 5 9 9 | 1 0 0 | Inf | 000 2 | 0 | 7 14 | MSPNDKVIMFVSQKHIADDLSSDFNIQGISAESLHGNSEQSDQERAVEDFKSGNIKILITTDIVSRGLDLNDVTHVYNY DFPRNIDVYVHRVGYIGRTGKTGTSVTLITQRDSKMAGELIKILDRANQSVPEDLVVMAEQYKLNQQKRHRETRSR |

EP 2 620 772 A1

(continued)

| No. /Seq. No. ID | Clone No | Gene symbol | Rating score | Sensitivity (Freq in GC), % | Specificity (100 - Freq in HD), % | Odds ratio | Fisher exact t test p-value | Freq. in inflammatory diseases, % K25 | K29 | Peptide sequence |
|---|---|---|---|---|---|---|---|---|---|---|
| 552 | 35 | MAGEC1// MA GEC2 | 7132 | 11594 | 0 | Inf | 0008 | 0 | 0 | LALIEVDPDDSYVFVNTLDLTSEGCLSDEQGMSQNRLLILILSIIFIKGTYASEEVIWDVLSGIGVRAGREHFAFGEPREL LTKVWVQEHYLEYREVPNSSPPRYEFLWGPRAHSESIKKKVLEFLAKLNNTVPSSFPSWYKDALKDVEERVQATIDT ADDATVMASESLSVMSSN |
| 6443 | 14 | HORMAD1 | 5469 | 3800 | 100 | Inf | 0063 | 0 | 102 | AVSVSCITYLRGIFPECAYGTRYLDDLCVKILREDKNCPGSTQLVKWMLGCYDALQKKYLRMVVLAVYTNPEDPQTI SECYQFKFKYTNNGPLMDFISKNQSNESSMLSTDTKKASIPLIRKIYILMQNLGPLPNDVCLTMKLFYYDEVTPPDYQP PGFKDGDCEGVIFEGEPMYLNVGEVSTPFHIFKVKVTTERERMENIDSTILSPKQIKTPFQKILRDKDVEDEQEHYTSD DLDIETKMEEQEKNPASSELEEPSLVCEEDEIMRSKESPDL |
| 7445 | 1 | PRKACA | 4584 | 5553 | 9953 | 8296 | 0022 | 0 | 816 | GSEQESVKEFLAKAKEDFLKKWESPAQNTAHLDQFERIKTLGTGSFGRVMLVKHKETGNHYAMKILDKQKVVKLKQ IEHTLNEKRILQAVNFPFLVKLEFSFKDNSNLY |

(continued)

| No./Seq. ID | Clone No | Gene symbol | Rating score | Sensitivity (Freq in GC), % | Specificity (100-Freq in HD), % | Odds ratio | Fisher exact test p-value | Freq. in inflammatory diseases, % K25 | Freq. in inflammatory diseases, % K29 | **Peptide** sequence |
|---|---|---|---|---|---|---|---|---|---|---|
| 8 | 1477 | LRRC50 | 345 | 399 | 99533 | 6397 | 0072 | 0 | 102 | GSAGRGGCKEEINDPKEICVGSSDTSYHSQQKQSGDNGSGGHFAHPREDREDRGPRMTKSSLQKLCKQHKLSAWSHPQFEK |
| 90** | 72 | JARID2//RNF12 | 3296 | 347 | 99006 | 3654 | 0 11 | 0 | 0 | EQEYWRLVEEKIQALPTVPVTEEHVGSGLECPVCKDDYALGERVRQLPCNHLFHDGCIVPWLEQHDSCPVCRKSLTGQNTATNPPGLTGVSFSSSSSSSS |
| 10 | 835 | NOL8 | 2914 | 13 | 100 | Inf | 025 | 0 | 714 | GTPWNEDCGKEKPEEIQDPAALTSDAEQPSGFTFSFF |

(continued)

| No./Seq. No. ID | Clon | Gene symbol | Rating score | Sensitivity (Freq in GC), % | Specificity (100-Freq in HD), % | Odds ratio | Fisher exact t test p-value | Freq. in inflammatory diseases, % K25 | K29 | Peptide sequence |
|---|---|---|---|---|---|---|---|---|---|---|
| 119*** | 126 | UBR2 | 2372 | 27 | 9953 | 5459 | 013 | 192 | 0 | SLIESLH |
| 125****** | 75 | ITGB4 | 1646 | 09 | 100 | Inf | 05 | 0 | 0 | PGPGAEGASPPRGGRGWQGHAPAHGPGCSWQP |

(continued)

| No. / Seq. No. ID | Clone No | Gene symbol | Rating score | Sensitivity (Freq in GC), % | Specificity (100-Freq in HD), % | Odds ratio | Fisher exact test p-value | Freq. in inflammatory diseases, % | | Peptide sequence |
|---|---|---|---|---|---|---|---|---|---|---|
| | | | | | | | | K25 | K29 | |
| 1379 | 14 | BIRC5 | 1575 | 08 | 1000 | Inf | 05 | 0 | 0 | MGAPTLPPAWQPFLKDHRISTFKNWPFLEGCACTPERMAEAGFIHCPTENEPDLAQCFFCFKELEGWEPDDDPIEEHKKHSSGCAFLSVKKQFEELTLGEFLKLDRERAKNKIAKETNNKKKEFEETAKKVRRAIEQLAAMD |

12

(continued)

| No./Seq. No. ID | Clone No | Gene symbol | Rating score | Sensitivity (Freq in GC), % | Specificity (100-Freq in HD), % | Odds ratio | Fisher exact test p-value | Freq. in inflammatory diseases, % | | Peptide sequence |
|---|---|---|---|---|---|---|---|---|---|---|
| | | | | | | | | K25 | K29 | |
| 14-114 ******* | | | 144 | 08 | 1000 | Inf | 0 5 | 1 92 | 0 | WLQEVTVEKLCV |

(continued)

| No./Seq. ID | Clone No | Gene symbol | Rating score | Sensitivity (Freq in GC), % | Specificity (100-Freq in HD), % | Odds ratio | Fisher exact test p-value | Freq. in inflammatory diseases, % | | Peptide sequence |
|---|---|---|---|---|---|---|---|---|---|---|
| | | | | | | | | K25 | K29 | |
| 1556 | 14 | TYR | 0.956 | 25.56 | 99.06 | 27.17 | 029 | 0 | 20.4 | TQYESGSMDKAANFSFRNTLEGFASPLTGIADASQSSMHNALHIYMNGTMSQVQGSANDPIFLLHHAFVDRCDLPQEEMSASYT |
| 1679 | 13 | ZNF282 | 0.853 | 13.53 | 99.53 | 26.93 | 063 | 0 | 20.4 | EQRHPEEREIPMDPEAGAEPLVPAQDASSQVKREDTLCVRGQRGLEERAIPTESITDSPISAQDLLSRIKQEEHQCVWDQQDLADRDIPTDPNSESLISAHDILSWIKQEEQPYPWGPRDSMDGELGLDSGPSDSLLMVKNPPP |
| 1759 | 14 | ARMC3 | 0.404 | 14.53 | 99.53 | 26.95 | 0 63 | 1.92 | 1.02 | CAGDELTANELCRLGALDILEEVNVSGTRKNKFSEAAYNNCSITIFP |
| 1836 | 14 | SPAG17 | 0.377 | 23.36 | 99.06 | 13.41 | 1 | 0 | 10.2 | NNLKLSVPDNRQLLEQESIMKAQPQHESLGNKGISKTEISDQEKEKEKEKIPFILEGSLKAWKEEQHRLAEEERLREEKKAEKKGKEAGKKKGKDNAEKEDSRSLKKKSPYKEKS |

| No. / Seq. No ID | Clone No | Gene symbol | Rating score | Sensitivity (Freq in GC), % | Specificity (100-Freq in HD), % | Odds ratio | Fisher exact test p-value | Freq. in inflammatory diseases, % K25 | K29 | Peptide sequence |
|---|---|---|---|---|---|---|---|---|---|---|
| 194 | 146 | DKFZP566 E164 | 0128 | 13 | 9953 | 2695 | 063 | 0 | 102 | EKPSKVSLKSSDRQGSDEESVHSDTRDLWTTTTLSQAQLNMPLSEVCEGFDEEGRNISKTRGWHSPGRGSLDEG |

(continued)

| No. / Seq. ID | Clone No | Gene symbol | Rating score | Sensitivity (Freq in GC), % | Specificity (100-Freq in HD), % | Odds ratio | Fisher exact t test p-value | Freq. in inflammatory diseases, % | | Peptide sequence |
|---|---|---|---|---|---|---|---|---|---|---|
| | | | | | | | | K25 | K29 | |
| 203 | 96 | RNF14 | 0.065 | 14.5 | 99.53 | 26.95 | 0.063 | 0.1 | 5.1 | VDVDDDIWEDEVED |

Footnotes to Table 2:

*- Generated by the translation of POP5 mRNA in Frame 3;

**- the first 26nts are from JARID2. the rest from RNF126.

***- the first 5 amino acids conform to 1214-1218 amino acids of UBR2, the rest come from a 12 nucleotide insertion before exon 43 containing translation STOP.

****- novel splice variant- alternative Intron 1 5' splice site - shorter exon 1, creating frameshift. The first 10 amino acids correspond to ITGB4 precursor protein sequence, the rest is 63% homologous to laminin, alpha 5, isoform CRA_b.

*****- Generated by the translation of the Homo sapiens cDNA clone MGC: 18304 IMAGE:4180012 (accession BC008618.1 GI:14250372); http://www.ncbi.nlm.nih.gov/nucleotide/14250372?report=genbank&log$=nuclalign&blast_rank= 1&RID=H0S3UY1S01S

Among the 20 antigens set forth in Table 2 there are proteins that have been previously described as immunogenic in cancer patients: proteins from the CTAG2, DDX53, HORMAD1, BIRC5, PRKACA, TYR, SPAG17 and MAGE families, however their individual use for autoantibody detection for gastric cancer diagnosis either doesn't reach an adequate sensitivity or has not been studied. For example, the most frequent spontaneous humoral response has been shown against CTAG2 (highly cross-reactive with CTAG1B/NYESO1) - observed in patients with melanoma, gastric, breast, lung, ovarian, liver and other cancers with a frequency of 2-20% [13-17], but this is not sufficient for the development of a cancer diagnosis test based on the detection of anti-CTAG2 antibodies.

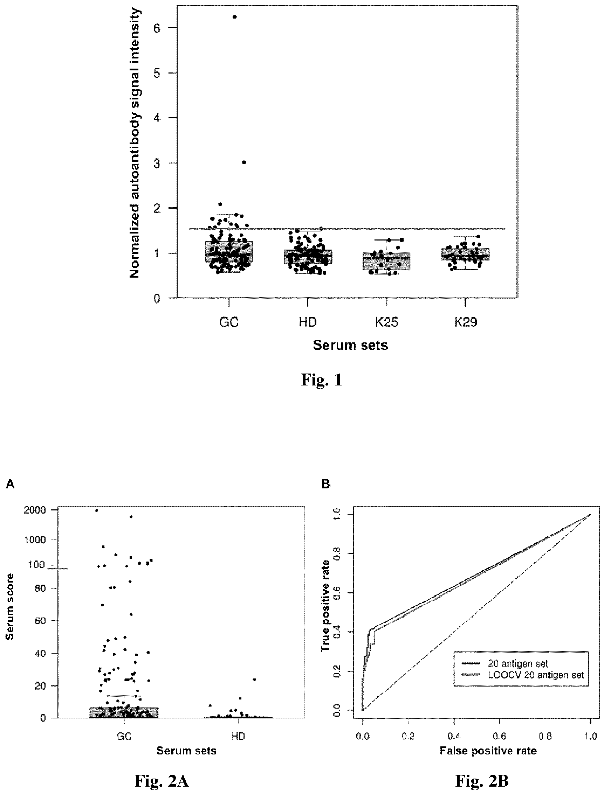

The phage display SEREX approach doesn't select for the correct open reading frame (ORF) of a cloned cDNA and can yield immunogenic peptides generated due to the translation of alternative ORFs and inserts of genomic DNA that most likely represent mimotopes of cancer antigens whose identity is elusive. The inventors describe here the identification of a novel peptide that forms as a results of frame 3 translation of POP5 cDNA - clone No 509, and shows a similar autoantibody frequency in gastric cancer as the previously known well-characterised cancer testis antigens CTAG2 and MAGEC1 (Table 2). Besides, this peptide is not recognised by any of the analysed healthy donor and gastric inflammatory disease patients' sera, yielding a Fisher's exact test p-value of 6.7x10-5. The autoantibody response against this peptide in the analysed cohorts of sera is shown in Fig 1.

*The diagnostic value of the autoantibody test.* The diagnostic value of the 20 antigen set was determined by the analysis of the antigen microarray using an independent validation set of sera, that comprised sera from 239 various stage GC patients and of various histological types and 213 healthy individuals with no history of cancer, 52 patients with peptic ulcer and 98 patients with acute or chronic gastritis and all the analyses and calculations were performed as described above. Results showed that the mean values of the antibody test serum score in cancer patient and healthy donor cohorts were 31 and 0.24, respectively. The difference reached statistical significance (P=$1.3\times10^{-20}$ Mann-Whitney U test) (Figure 2A) and the test discriminated cancer patients from age and gender matched healthy controls with a 42% sensitivity and 95% specificity, ROC curve AUC was 0.69, 95% CI 0.66-0.72. Crossvalidation analysis (Leave-one-out crossvalidation, LOOCV) showed AUC 0.68, 95% CI 0.65-0.71 (Figure 1B, Table 3). The diagnostic performance of this test is shown in Table 3.

Table 3. The diagnostic performance of the autoantibody test containing isolated antigenic peptides comprising amino acid sequences of SEQ ID Nos. 1 to 20.

| | GC vs HD | LOOCV GC vs HD | Stage 1 vs HD | GC vs K25 | GC vs K29 |
|---|---|---|---|---|---|
| AUC(95%CI) | 0.6921(0.6649 -0.7193) | 0.679(0.6512 -0.7068) | 0.7385(0.6722 -0.8047) | 0.6816(0.6421 -0.7211) | 0.5993(0.5569 -0.6417) |
| AUC SE | 0.01 | 0.01 | 0.03 | 0.02 | 0.02 |
| P value | 1.3E-20 | 1.1E-18 | 3.50E-015 | 0.0000018 | 0.0011 |
| Cutoff | 0.358 | 0.32 | 0.485 | 0.358 | 4.641 |
| Sn | 0.423 | 0.402 | 0.516 | 0.423 | 0.28 |
| Sp | 0.948 | 0.948 | 0.948 | 0.923 | 0.918 |
| OD | 13.44 | 12.328 | 19.588 | 8.783 | 4.382 |
| Accuracy | 0.67 | 0.659 | 0.893 | 0.512 | 0.466 |
| Abbreviations: LOOVC - Leave-one-out cross validation, AUC - area under the curve, SE - standard error, Sn - sensitivity, Sp - specificity, OD - odds ratio, PPV - positive predictive value, NPV - negative predictive value. | | | | | |

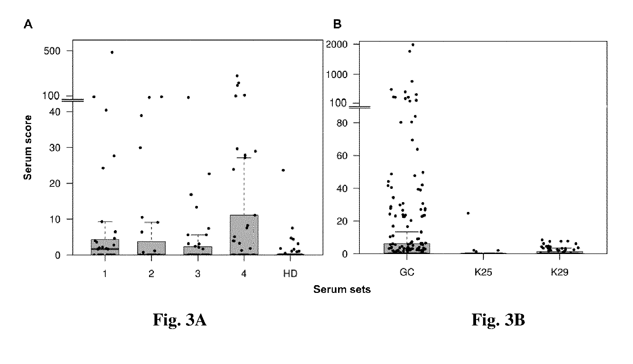

In order to determine the possibility to use this test for early gastric cancer detection, the results were compared between stage I and stage IV cancer patients. The difference did not reach statistical significance meaning that this test unlike other serological markers can detect early and late stage cancer with a similar efficiency and can be used for early diagnosis of gastric cancer (Table 3, Figure 3A). Thereto the test results score 1.1) were discriminated with a lower precision (Figure 3B, Table 3). Chronic gastritis has been shown to be a pre-cancerous lesion and it is possible that this group comprised yet undiagnosed early stage gastric cancer patients that could explain the diminished discrimination capacity of the 20 antigen set of this sample set.

*Use of the offered antigen set.* The offered antigen set can be used for the detection of gastric cancer specific autoantibodies in human serum for the diagnosis, prognosis and prediction of therapy outcome of gastric cancer. Any kind of high sensitivity antibody detection system like ELISA, Western blot, any technology based on beads or microarrays or any other material that is appropriate for a multiplex test can be used for this purpose. The identified antigens can be obtained either by chemical synthesis or by using any biotechnological approach for the generation and purification of recombinant proteins. The antigens can be bound to the above substrates that are marked with a fluorescent or any other informative signal that ensures the information about the identity of the bound antigen. The binding of antigens to substrates can be achieved through direct conjugation or through intermediate elements for example but not limited to StrepTag®, HaloTag® or other tags. The presence of a reactive antibody specific for the antigens can be detected by incubating the antigen-bound substrate with the serum sample of interest, washing away the unbound antibodies, and using an anti-human IgG secondary antibody that is marked with a reporter molecule that emits a signal detectable by appropriate machines.

**References cited**

1. Jemal A, Siegel R, Ward E, et al. Cancer statistics, 2008. CA Cancer J Clin. 2008;58(2):71-96.

2. Gospodarowicz M, Mackillop W, O'Sullivan B, et al. Prognostic factors in clinical decision making: the future. Cancer. 2001;91(8 Suppl):1688-1695.

3. Crew KD and Neugut Al. Epidemiology of gastric cancer. World J Gastroenterol. 2006;12(3):354-362.

4. Talley NJ, Silverstein MD, Agreus L, et al. AGA technical review: evaluation of dyspepsia. American Gastroenterological Association. Gastroenterology. 1998;114(3):582-595.

5. Sturgeon CM, Duffy MJ, Hofmann BR, et al. National Academy of Clinical Biochemistry Laboratory Medicine Practice Guidelines for use of tumor markers in liver, bladder, cervical, and gastric cancers. Clin Chem. 2010;56(6):el-48.

6. Kalnina Z, Silina K, and Line A. Autoantibody Profiles as Biomarkers for Response to Therapy and Early Detection of Cancer. Curr Cancer Ther Rev. 2008;4(2):149-156.

7. Tureci O, Sahin U, and Pfreundschuh M. Serological analysis of human tumor antigens: molecular definition and implications. Mol Med Today. 1997;3(8):342-349.

8. Cancer Immunome Database, [database online]. New York, NY: The Academy of Cancer Immunology, Ludwig Institute for Cancer Research; 1997.

9. Kalnina Z, Silina K, Meistere I, et al. Evaluation of T7 and lambda phage display systems for survey of autoantibody profiles in cancer patients. J Immunol Methods. 2008;334(1-2):37-50.

10. Wex T, Leodolter A, Bornschein J, et al. Interleukin 1 beta (IL1B) gene polymorphisms are not associated with gastric carcinogenesis in Germany. Anticancer Res. 2010;30(2):505-511.

11. Laxman B, Morris DS, Yu J, et al. A first-generation multiplex biomarker analysis of urine for the early detection of prostate cancer. Cancer Res. 2008;68(3):645-649.

12. Greiner M. Two-graph receiver operating characteristic (TG-ROC): update version supports optimisation of cut-off values that minimise overall misclassification costs. J Immunol Methods. 1996;191(1):93-94.

13. Gnjatic S, Nishikawa H, Jungbluth AA, et al. NY-ESO-1: review of an immunogenic tumor antigen. Adv Cancer Res. 2006;95:1-30.

14. Fujita S, Wada H, Jungbluth AA, et al. NY-ESO-1 expression and immunogenicity in esophageal cancer. Clin Cancer Res. 2004;10(19):6551-6558.

15. Sugita Y, Wada H, Fujita S, et al. NY-ESO-1 expression and immunogenicity in malignant and benign breast tumors. Cancer Res. 2004;64(6):2199-2204.

16. Reuschenbach M, von Knebel Doeberitz M, and Wentzensen N. A systematic review of humoral immune responses against tumor antigens. Cancer Immunol Immunother. 2009;58(10):1535-1544.

17. Stockert E, Jager E, Chen YT, et al. A survey of the humoral immune response of cancer patients to a panel of human tumor antigens. J Exp Med. 1998;187(8):1349-1354.

18. Koziol JA, Zhang JY, Casiano CA, et al. Recursive partitioning as an approach to selection of immune markers for tumor diagnosis. Clin Cancer Res. 2003; 9(14):5120-5126.

**Claims**

1. An isolated antigenic peptide comprising an amino acid sequence of SEQ ID No. 1 or a variant, homologue, or fragment thereof.

2. A kit for the detecting, diagnosing, prognosing, staging or monitoring gastric cancer, comprising the peptide according to claim 1.

3. A kit according to claim 2 containing isolated antigenic peptides comprising an amino acid sequence of SEQ ID Nos. 1 to 20.

**4.** A method for *in vitro* detection of antibodies against a peptide according to claim 1 comprising the steps:

    a) use of an isolated body fluid and a peptide according to claim 1,
    b) bringing in contact isolated body fluid and the peptide of step a), and
    c) determine, whether antibodies from isolated body fluid are bound to the peptide.

**5.** The method according to claim 4, wherein the isolated body fluid is contacted with a group of peptides comprising an amino acid sequence of SEQ ID Nos. 1 to 20.

**6.** Use of the kit according to any one of claims 2 or 3 for the detecting, diagnosing, prognosing, staging or monitoring gastric cancer.

Fig. 1

Fig. 2A

Fig. 2B

**Fig. 3A**

**Fig. 3B**

**EP 2 620 772 A1**

<table>
<tr><td colspan="2" rowspan="2" style="text-align:center">Europäisches Patentamt<br>European Patent Office<br>Office européen des brevets</td><td style="text-align:center">**EUROPEAN SEARCH REPORT**</td><td style="text-align:center">Application Number<br>EP 12 15 2404</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/068628 A1 (ABLYNX NV [BE]; HERMANS GUY [BE]; REVETS HILDE [BE]) 4 June 2009 (2009-06-04) * sequence 1495 *<br>----- | 1 | INV. G01N33/574 |
| X | Anonymous: "Homo sapiens (human) processing of precursor 5, ribonuclease P/MRP subunit (S. cerevisiae), isoform CRA_d", , 4 September 2011 (2011-09-04), XP55028154, Retrieved from the Internet: URL:http://www.ebi.ac.uk/ena/data/view/EAW98208 [retrieved on 2012-05-24] * the whole document *<br>----- | 1 | |
| X | EP 2 107 127 A1 (UNIV JOSEPH FOURIER [FR]; INST NAT SANTE RECH MED [FR]) 7 October 2009 (2009-10-07) * abstract * * paragraphs [0025], [0063], [0100]; sequence 180 *<br>----- | 1-6 | |

TECHNICAL FIELDS SEARCHED (IPC)

G01N

The present search report has been drawn up for all claims

| Place of search | Date of completion of the search | Examiner |
|---|---|---|
| The Hague | 24 May 2012 | Gundlach, Björn |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**                    EP 12 15 2404

This annex lists the patent family members relating to the patent documents cited in the above-mentioned European search report.
The members are as contained in the European Patent Office EDP file on
The European Patent Office is in no way liable for these particulars which are merely given for the purpose of information.

24-05-2012

| Patent document cited in search report | | Publication date | Patent family member(s) | | Publication date |
|---|---|---|---|---|---|
| WO 2009068628 | A1 | 04-06-2009 | AU | 2008328779 A1 | 04-06-2009 |
| | | | AU | 2008328781 A1 | 04-06-2009 |
| | | | AU | 2008328784 A1 | 04-06-2009 |
| | | | AU | 2008328785 A1 | 04-06-2009 |
| | | | CA | 2705890 A1 | 04-06-2009 |
| | | | CA | 2706200 A1 | 04-06-2009 |
| | | | CA | 2706425 A1 | 04-06-2009 |
| | | | CA | 2706675 A1 | 04-06-2009 |
| | | | CN | 101970490 A | 09-02-2011 |
| | | | EP | 2215123 A1 | 11-08-2010 |
| | | | EP | 2215125 A1 | 11-08-2010 |
| | | | EP | 2220120 A2 | 25-08-2010 |
| | | | EP | 2225278 A2 | 08-09-2010 |
| | | | JP | 2011504740 A | 17-02-2011 |
| | | | KR | 20100097716 A | 03-09-2010 |
| | | | US | 2011028695 A1 | 03-02-2011 |
| | | | US | 2011053865 A1 | 03-03-2011 |
| | | | US | 2011059090 A1 | 10-03-2011 |
| | | | US | 2011189203 A1 | 04-08-2011 |
| | | | WO | 2009068625 A2 | 04-06-2009 |
| | | | WO | 2009068627 A2 | 04-06-2009 |
| | | | WO | 2009068628 A1 | 04-06-2009 |
| | | | WO | 2009068630 A1 | 04-06-2009 |
| | | | WO | 2009068631 A1 | 04-06-2009 |
| EP 2107127 | A1 | 07-10-2009 | AU | 2009231511 A1 | 08-10-2009 |
| | | | EP | 2107127 A1 | 07-10-2009 |
| | | | JP | 2011517937 A | 23-06-2011 |
| | | | US | 2011059856 A1 | 10-03-2011 |
| | | | WO | 2009121878 A2 | 08-10-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JEMAL A ; SIEGEL R ; WARD E et al.** *Cancer statistics,* 2008
- **CA CANCER.** *J Clin.,* 2008, vol. 58 (2), 71-96
- **GOSPODAROWICZ M ; MACKILLOP W ; O'SULLIVAN B et al.** Prognostic factors in clinical decision making: the future. *Cancer,* 2001, vol. 91 (8), 1688-1695
- **CREW KD ; NEUGUT AL.** Epidemiology of gastric cancer. *World J Gastroenterol.,* 2006, vol. 12 (3), 354-362
- **TALLEY NJ ; SILVERSTEIN MD ; AGREUS L et al.** AGA technical review: evaluation of dyspepsia. American Gastroenterological Association. *Gastroenterology,* 1998, vol. 114 (3), 582-595
- **STURGEON CM ; DUFFY MJ ; HOFMANN BR et al.** National Academy of Clinical Biochemistry Laboratory Medicine Practice Guidelines for use of tumor markers in liver, bladder, cervical, and gastric cancers. *Clin Chem.,* 2010, vol. 56 (6), el-48
- **KALNINA Z ; SILINA K ; LINE A.** Autoantibody Profiles as Biomarkers for Response to Therapy and Early Detection of Cancer. *Curr Cancer Ther Rev.,* 2008, vol. 4 (2), 149-156
- **TURECI O ; SAHIN U ; PFREUNDSCHUH M.** Serological analysis of human tumor antigens: molecular definition and implications. *Mol Med Today,* 1997, vol. 3 (8), 342-349
- Cancer Immunome Database. The Academy of Cancer Immunology, 1997
- **KALNINA Z ; SILINA K ; MEISTERE I et al.** Evaluation of T7 and lambda phage display systems for survey of autoantibody profiles in cancer patients. *J Immunol Methods,* 2008, vol. 334 (1-2), 37-50
- **WEX T ; LEODOLTER A ; BORNSCHEIN J et al.** Interleukin 1 beta (IL1B) gene polymorphisms are not associated with gastric carcinogenesis in Germany. *Anticancer Res,* 2010, vol. 30 (2), 505-511

- **LAXMAN B ; MORRIS DS ; YU J et al.** A first-generation multiplex biomarker analysis of urine for the early detection of prostate cancer. *Cancer Res.,* 2008, vol. 68 (3), 645-649
- **GREINER M.** Two-graph receiver operating characteristic (TG-ROC): update version supports optimisation of cut-off values that minimise overall misclassification costs. *J Immunol Methods,* 1996, vol. 191 (1), 93-94
- **GNJATIC S ; NISHIKAWA H ; JUNGBLUTH AA et al.** NY-ESO-1: review of an immunogenic tumor antigen. *Adv Cancer Res.,* 2006, vol. 95, 1-30
- **FUJITA S ; WADA H ; JUNGBLUTH AA et al.** NY-ESO-1 expression and immunogenicity in esophageal cancer. *Clin Cancer Res.,* 2004, vol. 10 (19), 6551-6558
- **SUGITA Y ; WADA H ; FUJITA S et al.** NY-ESO-1 expression and immunogenicity in malignant and benign breast tumors. *Cancer Res.,* 2004, vol. 64 (6), 2199-2204
- **REUSCHENBACH M ; VON KNEBEL DOEBERITZ M ; WENTZENSEN N.** A systematic review of humoral immune responses against tumor antigens. *Cancer Immunol Immunother,* 2009, vol. 58 (10), 1535-1544
- **STOCKERT E ; JAGER E ; CHEN YT et al.** A survey of the humoral immune response of cancer patients to a panel of human tumor antigens. *J Exp Med.,* 1998, vol. 187 (8), 1349-1354
- **KOZIOL JA ; ZHANG JY ; CASIANO CA et al.** Recursive partitioning as an approach to selection of immune markers for tumor diagnosis. *Clin Cancer Res.,* 2003, vol. 9 (14), 5120-5126